(19) Europäisches Patentamt
European Patent Office
Office européen des brevets

(11) Veröffentlichungsnummer: **0 311 002**
**A2**

(12) **EUROPÄISCHE PATENTANMELDUNG**

(21) Anmeldenummer: **88116379.4**

(51) Int. Cl.⁴: **C07H 15/252 , A61K 31/70**

(22) Anmeldetag: **04.10.88**

Patentansprüche für folgende Vertragsstaaten: ES + GR.

(30) Priorität: **07.10.87 DE 3733885**

(43) Veröffentlichungstag der Anmeldung: **12.04.89 Patentblatt 89/15**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Berscheid, Hans Gerd, Dr.**
**Rheinlandstrasse 21**
**D-6231 Schwalbach(DE)**
Erfinder: **Fehlhaber, Hans-Wolfram, Dr.**
**Thomas-Mann-Strasse 5a**
**D-6270 Idstein/Taunus(DE)**

(54) **Neue Anthracyclin-Derivate, ein Verfahren zu ihrer Herstellung und ihre Verwendung als Arzneimittel.**

(57) Die vorliegende Erfindung betrifft Anthracyclin-Derivate der Formel I

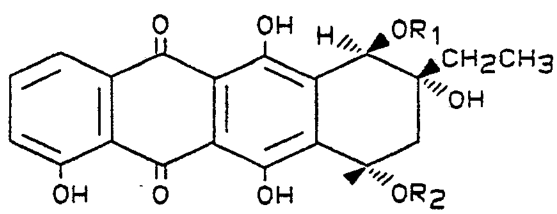

I

worin $R_1$ und $R_2$ verschieden sind und den Zuckerrest Roa (L-Rhodosamin) der Formel

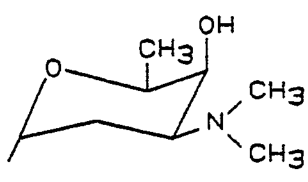

II

oder die Zuckerkombination -Roa-dF = D-Cin B (Roa: L-Rhodosamin; dF : 2-desoxy-L-Fucose; D-Cin B : D-Cinerulose B) der Formel III

EP 0 311 002 A2

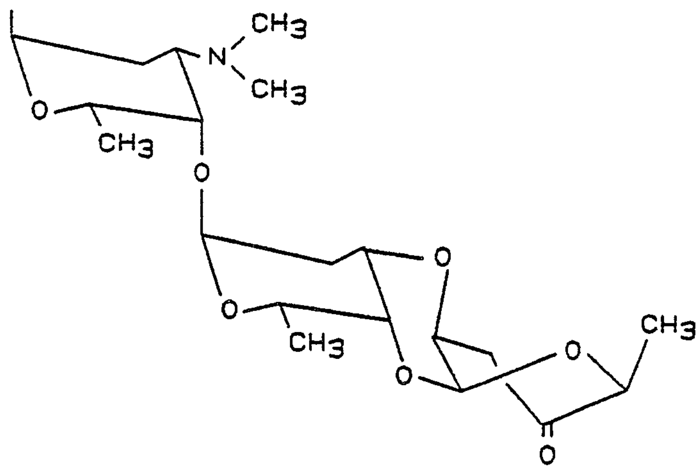

III

darstellen und ihre pharmazeutisch unbedenklichen Säureadditionssalze, sowie ein Verfahren zu ihrer Herstellung. Die erfindungsgemäßen Verbindungen haben cytostatische Wirkung und können daher als Arzneimittel verwendet werden.

# Neue Anthracyclin-Derivate,ein Verfahren zu ihrer Herstellung und ihre Verwendung als Arzneimittel

Die vorliegende Erfindung betrifft Anthracyclin-Derivate der Formel I

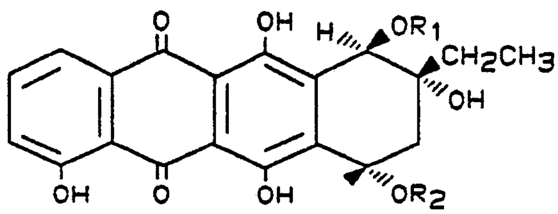

I

worin $R_1$ und $R_2$ verschieden sind und den Zuckerrest Roa (L-Rhodosamin) der Formel

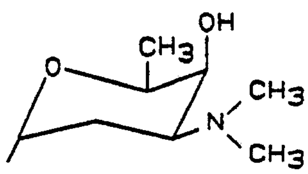

II

oder die Zuckerkombination -Roa-dF = D-Cin B (Roa: L-Rhodosamin; dF : 2-desoxy-L-Fucose; D-Cin B : D-Cinerulose B) der Formel III

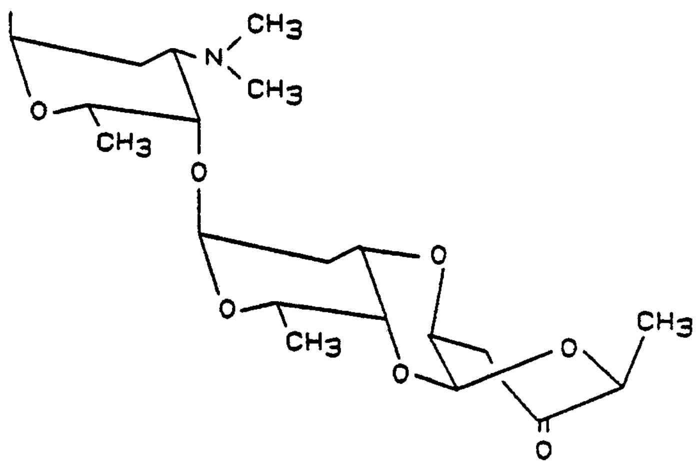

III

darstellen, sowie ihre pharmazeutisch unbedenklichen Säureadditionssalze.

Die Erfindung betrifft ferner ein Verfahren zur Herstellung der Verbindungen der Formel I, das dadurch gekennzeichnet ist, daß man die gemäß EP-A1-0-131 942 erhältlichen Verbindungen der Formel I, worin $R_1$ und $R_2$ verschieden sind und Roa (L-Rhodosamin) oder die Zuckerkombination Roa-dF = L-Cin B (Roa : L-Rhodosamin; dF : 2-desoxy-L-Fucose; L-Cin B : L-Cinerulose B) darstellen, epimerisiert. Das kann dadurch erreicht werden, daß man entweder a) die genannten gemäß EP-A1-0 131 942 erhältlichen Verbindungen direkt mit geeigneten basischen Reagenzien behandelt, oder b) das Rohgemisch, das man gemäß EP-A1-0 131 181 durch Kultivierung des Mikroorganismus-Stammes Y-11 472 (DSM 2658), Extraktion des Mycels und des Kulturfiltrats mit einem organischen Lösungsmittel und Behandlung des Rückstands mit einer verdünnten starken oder mittelstarken Säure gemäß EP-A1-0 131 942 erhält, an Kieselgel, das auf einen pH-Wert von 6,5-8,0 eingestellt wurde, chromatographiert.

Nach dem unter a) genannten Verfahren behandelt man die Ausgangsverbindungen ("Cytorhodin S" oder "Cytorhodin T") mit Lösungsmittelgemischen, die anorganische Basen wie Alkalimetallhydroxide, z.B. NaOH, oder basische Ammoniumsalze, wie z.B. Ammoniumacetat oder organische Basen wie z.B. Triäthylamin oder Tetraäthylammoniumhydroxid, einzeln oder auch in Mischung enthalten. Durch übliche chromatographische Methoden, z.B. Säulenchromatographie an Kieselgel, das auf einen pH-Wert von 6,5-8,0, vorzugsweise 8,0 eingestellt worden ist, können die erfindungsgemäßen Verbindungen voneinander getrennt und gereinigt werden.

Nach der unter b) genannten Verfahrensvariante wird das gemäß EP-A1-0 131 942 durch Säurebehandlung gewonnene Hydrolyseprodukt mit Lösungsmittelgemischen ohne Säurekomponente chromatographiert, vorzugsweise an mit Säure gewaschenen und mit einer Alkalimetallhydroxidlösung, z.B. Natriumhydroxidlösung auf einen pH-Wert von 6,5-8,0, bevorzugt 7,0-7,5, eingestelltem Kieselgel, wobei die erfindungsgemäßen Verbindungen isoliert werden.

Man erhält durch das erfindungsgemäße Verfahren zum Beispiel die folgende Verbindung:

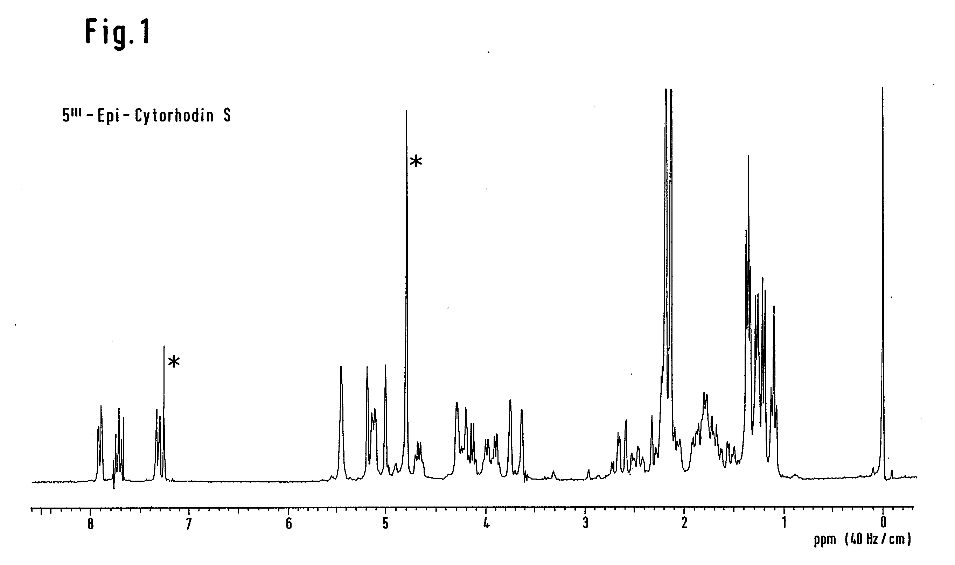

5‴-Epi-Cytorhodin S:
Rote, amorphe Substanz, leicht löslich in Methanol, Ethylacetat, Chloroform und Toluol, unlöslich in Wasser und Hexan.
Dünnschichtchromatographie: Kieselgel $60F_{254}$ "Merck"
System: Chloroform/Methanol/Eisessig/Wasser = 70/20/10/2,
$R_F$ = 0,54
UV-Maxima: 235, 255 (Sch ), 290, 495 nm in Methanol/10 % 1N HCl
$^1$H-NMR-Spektrum: Fig. 1
$C_{48}H_{64}N_2O_{17}$, M ber 940 (FAB-MS bestätigt)

Das 5‴-Epi-Cytorhodin S besitzt die Formel I, worin $R_1$ = L-Roa und $R_2$ = L-Roa-L-dF = D-Cin B bedeuten.

Die so hergestellten Verbindungen der Formel I zeichnen sich durch hoher cytostatische Wirkung und geringe Toxizität, insbesondere Nephrotoxizität aus, und können daher verwendet werden bei der Behandlung von Krebserkrankungen z.B. zur Therapie von malignen Tumoren.

Die cytostatische Wirkung der erfindungsgemäßen Verbindungen wurde im Zellproliferationstest an drei verschiedene Tumorzelllinien, wie im folgenden beschrieben, ermittelt:

L1210, A 549 bzw. HT 29 Tumorzellen in exponentieller Wachstumsphase werden in RPMI 1640 in einer Zelldichte von $5x10^3$ Zellen/ml in einer 96 Well Mikrotiterplatte für 72 Stunden (37 °C, 5 % $CO_2$, 95 % relative Luftfeuchte) mit unterschiedlichen Konzentrationen der Testsubstanz inkubiert. Als Kontrolle dienen Zellen, die anstelle der Testsubstanz mit frischem Medium inkubiert werden. Für jede Substanzkonzentration sowie für die Kontrolle werden jeweils 4fach-Bestimmungen angesetzt. Nach 65 Stunden werden 50 µl einer MTT-Lösung (MTT: 3-(4,5-Dimethylthiazolyl)-2,5-diphenyl-tetrazolium-bromid) in einer Konzentration von 2,5 mg/ml (gelöst in PBS) zugegeben. MTT wird durch lebende Zellen zu einem roten unlöslichen Formazan reduziert. Nach 7-24 Stunden Inkubation mit MTT (abhängig von der Art der verwendeten Zelle) wird das überstehende Medium sorgfältig abgesaugt. Der zunächst unlösliche Formazanfarbstoff wird durch Zugabe von 100 µl DMSO zu jedem Well, gefolgt von vorsichtigem Schütteln, gelöst. Die Extinktion der entstehenden Farblösung wird mit Hilfe eines Multiscan Photometers 340 CC der Firma Flow bei einer Wellenlänge von 492 nm ermittelt.

Die Ergebnisse werden als Verhältnis der Extinktionen nach Inkubation mit Testsubstanz, bezogen auf die unbehandelte Kontrolle, angegeben. Der Variationskoeffizient für wiederholte Bestimmungen ist kleiner als 15 %.

Für 5‴-Epi-Cytorhodin S wurden z.B. die folgenden Werte ermittelt:

| $IC_{50}$ (µg/ml) | | |
|---|---|---|
| L 1210 | HT 29 | A 549 |
| $8x10^{-3}$ | $2,1x10^{-3}$ | $3,4x10^{-3}$ |

Die Verbindungen und ihre Säureadditionssalze können daher als Medikamente zur Behandlung von Tumoren verwendet werden. Die Verbindungen können auf verschiedene Weise in Abhängigkeit von der Dosierungsform verabreicht werden.

Normalerweise werden die Verbindungen oder ihre Säureadditionssalze, z.B. mit D-Gluconsäure, mit pharmazeutisch üblichen Trägerstoffen oder Verdünnungsmitteln vermischt verabreicht. So können sie z.B. einzeln oder in Mischung zusammen mit Trägerstoffen wie Maltose oder Lactose oder als nicht-toxische Komplexe, z.B als Desoxyribonukleinsäure-Komplex, verabreicht werden.

Eine typische Verabreichungsart ist die Injektion einer Lösung der erfindungsgemäßen Verbindung in destilliertem Wasser oder in physiologischer Kochsalzlösung. Die Lösungen können intraperitoneal, intravenös oder intraarteriell injiziert werden.

Tagesdosis und Einheitsdosis können aus Tierversuchen und auch aus in vitro-Tests in der Weise festgesetzt werden, daß die Gesamtdosis, die kontinuierlich oder in Abständen verabreicht wird, einen vorher festgelegten Bereich nicht überschreitet. So beträgt die Gesamtdosis für einen Behandlungszyklus etwa 0,5-5 mg/kg Körpergewicht. Diese Dosis kann in entsprechenden Bruchteilen uber einen Zeitraum von 7 Tagen verabreicht werden. Es ist jedoch klar, daß konkrete Dosen für die Behandlung von Mensch oder Tier individuell festgelegt werden können in Abhängigkeit von der jeweiligen Situation des Patienten, z.B. Alter, Körpergewicht, Geschlecht, Empfindlichkeit, Nahrung, Zeitpunkt der Verabreichung, weiteren verabreichten Medikamenten, körperlichem Zustand des Patienten und Schwere der Erkrankung.

Die Herstellung der erfindungsgemäßen Verbindungen wird in den folgenden Beispielen beschrieben, wobei die Identifizierung der Komponenten unter den im folgenden beschriebenen Meßbedingungen erfolgte:

Die Protonen-Resonanzspektren ($^1$H-NMR-Spektren) werden auf einem HX-270 BRUKER Fourier-Transform-Kernresonanzspektrometer bei 270 MHz gemessen. Die Konzentrationen betrugen 2-4 mg/0,5 ml 99,8 % CDCl3; die Lösungen wurden sofort nach Herstellung mit 0,1 ml 5 % $Na_2CO_3$ in 99,5 % $D_2O$ geschüttelt.

Die in den Abbildungen mit einem Stern versehenen Signale rühren her von niedermolekularen Verunreinigungen im %o Bereich und von Lösungsmittelresten.

Die Massenspektren wurden auf dem Massenspektrometer MS-902 S, AEI, unter Verwendung einer FAB-(Fast-Atom-Bombardment)-Ionenquelle gemessen. Die Substanzen wurden in einer Matrix von Thioglycerin in die Ionenquelle eingebracht, teilweise unter Zusatz von Ammoniumchlorid.

Die Absorptionsspektren wurden im Bereich 200-700 nm gemessen in:

a) Wasser-Methanol 1:9
b) 10 % 1 n HCl in Methanol
c) 10 % 1 n NaOH in Methanol

Die Substanzkonzentration betrug 10-30 mg/l; angegeben werden die Absorptionsmaxima in nm.

**Beispiel 1:** Hydrolyse

750 g rohes basisches Glykosidgemisch aus einer Fermentation mit Streptomyces purpurascens DSM 2658 (entsprechend EP-A-0 131 942) wurden in 4 l 0,1 N HCl bei 20 °C unter Rühren gelöst und mit 2 N HCl der pH-Wert auf 1,1 eingestellt. Nach 16 Stunden wurde die Reaktionslösung mit 5 N NaOH auf pH 1,8 gebracht und 3mal mit je 6 l Toluol ausgeschüttelt; danach wurde mit 2 N NaOH der pH-Wert nacheinander auf pH 3, 4, 5 und 7 gestellt und die Lösung bei diesen pH-Werten jeweils 5 mal mit je 2 l $CHCl_3$ extrahiert. Nach dem Eindampfen im Vakuum lieferte der pH-5 Extrakt einen Rückstand von 81,6 g.

**Beispiel 2:** Chromatographie des Hydrolyseproduktes an basischem Kieselgel

15 g des in Beispiel 1 beschriebenen Hydrolyseproduktes wurden in 50 ml eines Gemisches von $CHCl_3$/Methanol/Wasser = 130/40/50 (Unterphase) gelöst und auf eine Stahlsäule (70 x 6 cm, gepackt mit 1,1 kg Kieselgel vom pH 8,0/20-45 µ, 60 A, Fa. Grace) gegeben und bei einem Fluß von 15 ml/min unter Druck mit der obigen Mischung chromatographiert. Fraktionen von 15 ml wurden aufgefangen, mit DC (s.o.) bewertet und in geeigneter Weise zusammengefaßt. Nach Eindampfen im Vakuum wurden folgende Ausbeuten erhalten:

| Fraktion | g | DC |
|---|---|---|
| 1-59 | 1,9 | |
| 60-80 | 1,5 | |
| 81-100 | 2,4 | v.a. 5'''-Epi-Cytorhodin S und Cytorhodin S |
| 101-145 | 1,7 | Cytorhodin S |
| 146-250 | 3,8 | |
| zum Ende | 1,2 | |
| mit 7 l Methanol | | |

**Beispiel 3:** Chromatographie an "reversed phase"-Material

2 g eines Gemisches von Cytorhodin S und 5'''-Epi-Cytorhodin S wurden in 90 ml einer Lösung von 0,5 % (v/v) Triäthylamin in Wasser (mit $H_3PO_4$ auf pH 3,0 gestellt) gelöst, und auf eine Stahlsäule (250 x 32 mm, unter Druck mit 110 g Umkehrphase Lichrosorb RP-18, 10 $\mu$, (Merck) gefüllt), gegeben und mit 2 l (dann 3 l) der mobilen Phasen 10 (dann 7) % (v/v)-Triäthylamin in Wasser (pH 3,0 mit $H_3PO_4$)/Acetonitril = 84/16 unter Druck chromatographiert. Bei einem Fluß von 10 ml/min wurden nach einem Vorlauf von 1,5 l Fraktionen von 10 ml gesammelt. Nach Zugabe vom 2 N NaOH bis zum pH 7,5 wurde mit $CHCl_3$ extrahiert, die Fraktionen mit DC geprüft und in geeigneter Weise vereinigt. Nach Eindampfen i.V. wurden folgende Produkte erhalten:

| Fraktion | mg | DC |
|---|---|---|
| 1-59 | 40 | polare Produkte |
| 60-160 | 375 | hauptsächlich Epi-Cytorhodin S |
| 161-304 | 860 | hauptsächlich Cytorhodin S |
| 305-320 | 460 | violettes Gemisch, Rf ähnlich Cytorhodin S |

Wiederholung der Chromatographie mit dem Produkt aus Fraktion 60-160 und der mobilen Phase mit 7 % (v/v)-Triäthylamin in der beschriebenen Weise führte zu insgesamt 97 mg 5'''-Epi-Cytorhodin S mit einer Reinheit von über 85 % nach $^1$H-NMR-Spektrum.

## Ansprüche

1. Verbindungen der Formel I

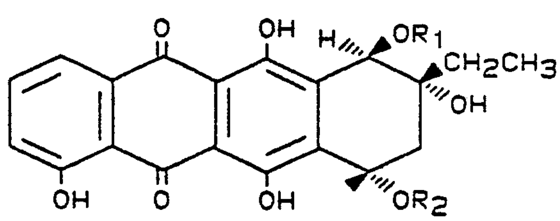

I

worin $R_1$ und $R_2$ verschieden sind und den Zuckerrest Roa: (L-Rhodosamin) der Formel

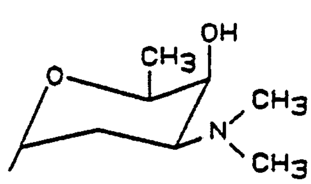

I I

oder die Zuckerkombination Roa-dF = D-Cin B (Roa: L-Rhodosamin; dF : 2-Desoxy-L-fucose; D-Cin B : D-Cinerulose B) der Formel III

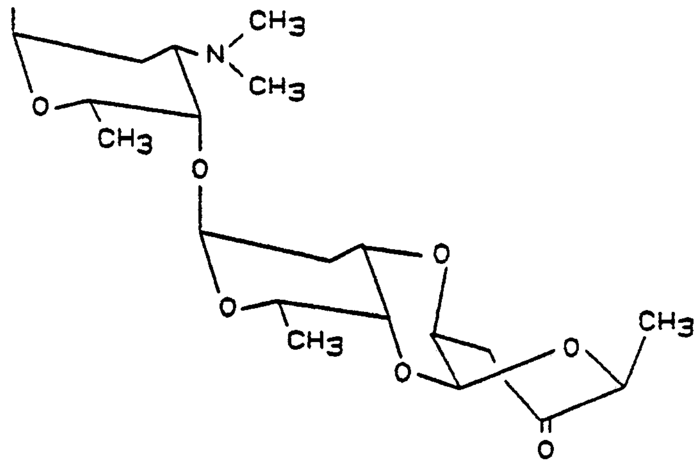

III

darstellen, sowie ihre pharmazeutisch unbedenklichen Säureadditionssalze.

2. Verbindungen der Formel I gemäß Anspruch 1, worin $R_1$ den Zuckerrest Roa und $R_2$ die Zuckerkombination Roa-dF = D-Cin B darstellt sowie deren pharmazeutisch unbedenkliche Säureadditionssalze.

3. Verbindung der Formel I gemäß Anspruch 1, worin $R_1$ die Zuckerkombination Roa-dF = D-Cin B und $R_2$ den Zuckerrest Roa darstellt.

4. Verfahren zur Herstellung von Verbindungen gemäß Anspruch 1, dadurch gekennzeichnet, daß man eine Verbindung der Formel I, worin $R_1$ und $R_2$ verschieden sind und den Zuckerrest Roa oder die Zuckerkombination Roa-dF = Cin B bedeuten, wobei Cin B die L-Cinerulose B darstellt, epimerisiert.

5. Verfahren gemäß Anspruch 4, dadurch gekennzeichnet, daß man entweder a) die genannten Ausgangsstoffe direkt mit basischen Reagenzien behandelt, oder b) das Gemisch, das gemäß EP-A1-0 131 181 durch Kultivierung des Mikroorganismus Y-11 472 (DSM 2658), Extrahieren des Mycels und des Kulturfiltrats mit einem organischen Lösungsmittel und anschließende Behandlung des erhaltenen Rückstandes mit einer verdünnten starken oder mittelstarken Säure gemäß EP-A1-0 131 942 erhalten wird, an auf einen pH-Wert von 6,5-8,0 eingestelltem Kieselgel chromatographiert.

6. Verfahren gemäß Anspruch 5, dadurch gekennzeichnet, daß die basischen Reagentien Alkalimetallhydroxide, basische Ammoniumsalze oder organische Basen sind.

7. Verfahren gemäß Anspruch 5, dadurch gekennzeichnet, daß das Kieselgel mit Natriumhydroxidlösung auf einen pH-Wert von 7 bis 7,5 eingestellt wird.

8. Arzneimittel, gekennzeichnet durch einen Gehalt an einer Verbindung gemäß Anspruch 1.

9. Verwendung einer Verbindung gemäß Anspruch 1, zur Herstellung eines Arzneimittels mit cytostatischer Wirkung.

Patentansprüche für folgende Vertragsstaaten: ES und GR

1. Verfahren zur Herstellung von Verbindungen der Formel I

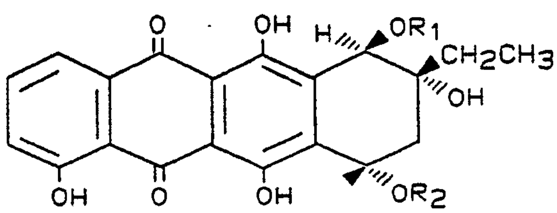

I

worin $R_1$ und $R_2$ verschieden sind und den Zuckerrest Roa (L-Rhodosamin) der Formel

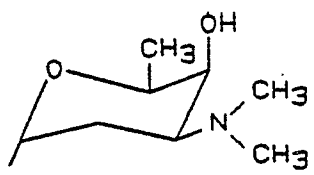

II

oder die Zuckerkombination -Roa-dF = D-Cin B (Roa: L-Rhodosamin; dF : 2-Desoxy-L-fucose; D-Cin B : D-Cinerulose B) der Formel III

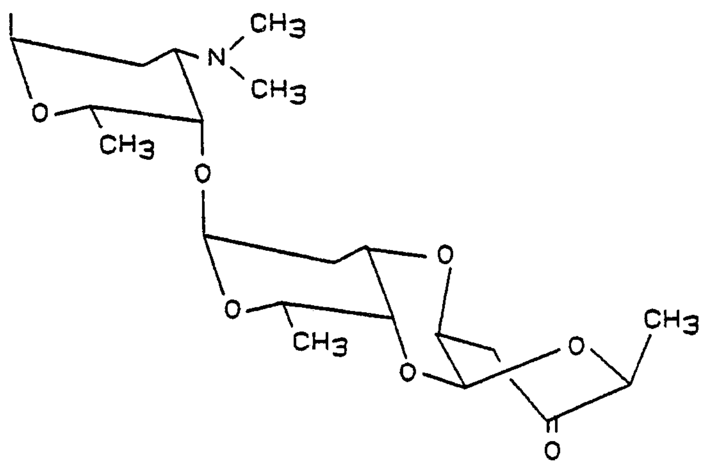

III

darstellen, sowie deren pharmazeutisch unbedenklichen Säureadditionssalze, dadurch gekennzeichnet, daß man eine Verbindung der Formel I, worin $R_1$ und $R_2$ verschieden sind und den Zuckerrest Roa oder die Zuckerkombination Roa-dF = Cin B bedeuten, wobei Cin B die L-Cinerulose B darstellt, epimerisiert.

2. Verfahren gemäß Anspruch 4, dadurch gekennzeichnet, daß man entweder a) die genannten Ausgangsstoffe direkt mit basischen Reagenzien behandelt, oder b) das Gemisch, das gemäß EP-A1-0 131 181 durch Kultivierung des Mikroorganismus Y-11 472 (DSM 2658), Extrahieren des Mycels und des Kulturfiltrats mit einem organischen Lösungsmittel und anschließende Behandlung des erhaltenen Rückstandes mit einer verdünnten starken oder mittelstarken Säure gemäß EP-A1-0 131 942 erhalten wird, an auf einen pH-wert von 6,5-8,0 eingestelltem Kieselgel chromatographiert.

3. Verfahren gemäß Anspruch 2, dadurch gekennzeichnet, daß die basischen Reagentien Alkalimetallhydroxide, basische Ammoniumsalze oder organische Basen sind.

4. Verfahren gemäß Anspruch 2, dadurch gekennzeichnet, daß das Kieselgel mit Natriumhydroxidlösung auf einen pH-Wert von 7 bis 7,5 eingestellt wird.

5. Verfahren gemäß Anspruch 1, dadurch gekennzeichnet, daß man eine Verbindung der Formel I darstellt, worin $R_1$ den Zuckerrest Roa und $R_2$ die Zuckerkombination Roa-dF = D-Cin B darstellt.

6. Verfahren gemäß Anspruch 1, dadurch gekennzeichnet, daß man eine Verbindung der Formel I darstellt, worin $R_1$ di Zuckerkombination Roa-dF = D-Cin B und $R_2$ den Zuckerrest Roa darstellt.

7. Verwendung einer Verbindung gemäß Anspruch 1 zur Herstellung eines Arzneimittels mit cytostatischer Wirkung.

Fig. 1

5'''-Epi-Cytorhodin S